# EUROPÄISCHE PATENTSCHRIFT

(11) **EP 2 364 688 B1**
(45) Veröffentlichungstag und Bekanntmachung des Hinweises auf die Patenterteilung: **24.07.2019**
(21) Anmeldenummer: 11157340.8
(22) Anmeldetag: 08.03.2011
(51) Int. Cl.: A61K 8/06, A61K 8/37, A61K 8/39, A61Q 19/00, A61K 8/36, A61K 8/04, A61K 8/86, A61K 8/81

(54) **Kosmetische Zubereitungen mit hoch-elastischer Textur**
Cosmetic preparations with highly elastic texture
Préparations cosmétiques dotées d'une texture hautement élastique

(30) Priorität: 12.03.2010 DE 102010011335
(43) Veröffentlichungstag der Anmeldung: 14.09.2011
(73) Patentinhaber: Beiersdorf AG, 20253 Hamburg (DE)
(72) Erfinder: Kröpke, Rainer, 22869, Schenefeld (DE); Lüttig, Kaja, 20255, Hamburg (DE); Kallmayer, Volker, Dr., 22393, Hamburg (DE)

(56) Entgegenhaltungen:
- EP-A2- 1 561 450
- EP-A2- 1 872 770
- WO-A1-03/017952
- DE-A1-102004 014 617
- DATABASE GNPD [Online] MINTEL; Dezember 2001 (2001-12), Neutrogena: "Anti-Cellulite Treatment Gel", XP002737178, Database accession no. 10098099
- DATABASE GNPD [Online] MINTEL; November 2007 (2007-11), Happy Bath Day-Precious Rose: "Milky Body Gel", XP002737179, Database accession no. 813693

## Beschreibung

Die Erfindung umfasst kosmetische oder dermatologische Zubereitung auf Basis einer O/W-Emulsion umfassend ein oder mehrere Stearinsäure-Emulgatoren und ein oder mehrere Polyacrylsäure-Verdicker im Verhältnis 10:1 auf. Die Zubereitungen weisen eine hoch elastische Textur bei geringer Viskosität auf.

Als kosmetische oder medizinische Zubereitungen sind oftmals Emulsionen, hier insbesondere W/O-, O/W- oder W/O/W-Emulsionen, im Einsatz. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten (W/O) in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Der Stand der Technik kennt mehrere wesentliche Faktoren, die einen positiven Einfluss auf die Stabilität von Emulsionen haben:
1. feinste Verteilung der Phasen ineinander: Je feiner die eine Phase in der anderen verteilt ist, je kleiner damit die dispergierten Teilchen sind, umso stabiler ist die Emulsion.
2. möglichst geringer Unterschied in der Dichte der beiden Phasen,
3. ein ausgewogenes Phasenvolumenverhältnis.

Mehrkomponentensysteme wie Lösungen, Emulsionen oder Suspensionen werden häufig aus ökonomischen oder anwendungstechnischen Gründen oder aus Stabilitätsgründen auf höhere Viskositäten eingestellt bzw. verdickt. So kann z.B. durch Erhöhung der Viskosität der externen oder internen Phase von Emulsionen oder Suspensionen erreicht werden, dass die Zeit bis zur Entmischung der Komponenten eines solchen Systems deutlich verlängert werden kann, was sich in einer Verlängerung der Lagerzeit bemerkbar macht. Durch Erhöhung der Viskosität wird auch bei vielen Produkten deren gleichmäßige Verteilbarkeit insbesondere auf unebenen Flächen verbessert. Dies gilt insbesondere für Hautpflegemittel und pharmazeutische Salben auf der Haut.

Das Temperaturverhalten der Polymere ist dabei eine wichtige Eigenschaft. Im Allgemeinen zeigen Polymere bei niedrigen Temperaturen eine hohe Viskosität, und bei hohen Temperaturen eine niedrige Viskosität. Erwünscht sind oftmals aber solche Polymere, die oberhalb bestimmter Temperaturen verdickend wirken, aber bei niedrigen Temperaturen in Lösung pumpbar und verarbeitbar bleiben, d.h. auch anwendungsfreundlich sind.

Bekannt als derartige Verdicker sind beispielsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine.

In DE 100 65 047 werden Gelcremes mit Ammoniumacryloyldimethyltaurat/ Vinylpyrrolidoncopolymeren beschrieben, deren Emulgatorgehalt unter 5% liegen muss. Doch solche Zubereitungen sind mithin gut bekannt und zeigen beim Verteilen nur einen Gelcharakter und lassen nur geringe Lipidkonzentrationen zu.

In der DE 19805918 sind lipidreduzierte Emulsionen und Emulgatorgele beschrieben, allerdings mit einem sehr geringen Anteil an Hautbefeuchtern (max. 3% Glycerin).

Die Schrift DE 10150734 offenbart Zubereitungen mit einem Gehalt an Dioic Acid. WO 01/51013 offenbart die Verwendung von mikrobiell verkapselten kosmetischen Inhaltsstoffen in kosmetischen Zubereitungen.

EP 1249232 offenbart ein Durch-die Haut Freisetzungsmaterial, dass polymere Partikel umfasst.

Wünschenswert ist es Zubereitungen zur Verfügung zu stellen, die eine viskoelastische Textur aufweisen bei vorteilhaft geringem Emulgatoranteil.

Der Stand der Technik offenbart zum Thema geringem Emulgatorgehalt und elastischem Verhalten jedoch dem Fachmann keinen Hinweis, der ihn zu der erfindungsgemäßen Zubereitungen führen würde.

Überraschenderweise wurde gefunden, dass eine bestimmte Kombination aus ein oder mehreren Stearinsäure-Emulgatoren und ein oder mehreren Polyacrylsäure-Verdicker, wobei das Verhältnis von Stearinsäure-Emulgatoren zu Polyacrylsäure-Verdicker im Bereich 10: 1 bis 1:1, insbesondere 3 : 1 liegt, der Zubereitung auf Basis einer O/W Emulsion zu einer hoch elastischen Textur bei geringer Viskosität führt.

Der Viskositätsbereich der erfindungsgemäßen Zubereitungen liegt zwischen 12.000 bis 100.000 mPas, insbesondere 12.000 bis 65.000 mPas, ganz besonders im Bereich von 12.000 bis 20.000 mPas.

Die erfindungsgemäße Viskosität wurde mit Hilfe eines Viscotester VT-02, der Firma Haake bei 25°C mit Hilfe des Drehkörpers 1 bzw. 2 und Ablesung der Skala 1 bzw. 2 bestimmt (entspricht einer Scherrate von 10 Pa/s).

Die erfindungsgemäßen Stearinsäure - Emulgatoren werden gewählt aus Stearinsäure (Pristerene 4960, Prifrac 2981 von Croda), Glycerylstearatcitrat (z.B. Imwitor 372 P, Fa. Sasol), Polyglyceryl-3 Methylglucose Distearate (Tego Care 450 von Evonik Goldschmidt), Polyglyceryl-3 Distearat (Cremophor GS 32, Fa. BASF), Steareth-21 (Tego Alkanol S 21 von Evonik Goldschmidt oder Brij 721 P von Croda), Steareth-2 (Tego Alkanol S2 von Evonik Goldschmidt oder Brij 72 von Croda), PEG-40 Stearat (Tego Acid S 40 P von Evonik Goldschmidt oder Myrj S40-PA-(WL) von Croda) und/oder Glyceryl Stearate + PEG-100 Stearate (Arlacel 165 von Croda). In Klammern sind die Handelsnamen und Herstellerfirmen der Emulgatoren genannt.

Der Anteil an Stearinsäure - Emulgatoren wird erfindungsgemäß auf maximal 1,5 Gew.%, bezogen auf die Gesamtmasse der Zubereitung, begrenzt.

Damit wird zum Einen die erfindungsgemäß vorteilhafte Textur erreicht und zum Anderen werden die Anteile an Emulgatoren verringert, was bei den Zubereitungen des Standes der Technik nicht oder nur mit Problemen möglich ist.

Als Polyacrylsäure-Verdicker werden bevorzugte Acrylates/C10-30 Alkyl Acrylate Crosspolymer eingesetzt.

Acrylates/C10-30 Alkyl Acrylate Crosspolymer ist ein Copolymer von C10-30 Alkyacrylat und ein oder mehrere Monomeren der Acrylsäure, Methacrylsäure und deren einfacher Ester verzweigt mit einem Allylether von Sucrose oder einem Allylether von Pentaerythritol . Dieses Polymer ist unter Acritamer 501ED (Rita), Acritamer 505ED (Rita), Aqupec HV-501ER (Sumitomo Seika Chemicals Co.), Carbopol ETD 2020 Polymer (Noveon), Carbopol 1342 Polymer (Noveon), Carbopol 1382 Polymer (Noveon), Carbopol Ultrez 20 Polymer (Noveon), Carbopol Ultrez 21 Polymer (Noveon), Pemulen TR-1 Polymer (Noveon), Pemulen TR-2 Polymer (Noveon) im Handel erhältlich.

Der Anteil an Polyacrylsäure Verdicker, wie insbesondere Acrylates/C10-30 Alkyl Acrylate Crosspolymer, liegt vorteilhaft im Bereich von 0,05 bis 1 Gew.%, bezogen auf die Gesamtmasse der Zubereitung. Insbesondere liegt der Anteil an an Polyacrylsäure Verdicker im Bereich um 0,3 Gew.%.

Die hoch elastische Textur bei geringer Viskosität, umgangssprachlich als Wobbeligkeit bezeichnet, äußerst sich in einer für den Anwender äußerst angenehmen Applikations- und Anwendungsweise.

Die erfindungsgemäßen Zubereitungen haben zunächst bei Applikation eine Textur ähnlich der von Götterspeise. D.h. sie sind stabil und können Kräfte und Schwingungen aufnehmen. Beim Verteilen auf der Haut wird die Textur jedoch flüssig und damit verreibbar.

Die hoch elastische Textur wird über das Verhältnis von viskosem zu elastischem Anteil über tan δ definiert.

Die Textureigenschaft von dispersen Zubereitungen, deren tan δ im Bereich von 0,05 bis 0,25 liegt (tan δ = G" : G' = viskoser Anteil: elastischer Anteil), zeigt die als Wobbeligkeit gekennzeichnete Eigenschaft.

Zur Ermittlung der Textureigenschaften über den tan δ werden die Zubereitungen zerstörungsfrei vermessen, in dem mit einem Platte/Platte-Rheometer, die Textur in Schwingungen versetzt wird (der Wert dieser Schwingungen wird in rad/s abgebildet (Omega = ω, ω stellt ein Maß der Belastung, Schwingung auf das System dar)). Die Antwort des Systems auf diese Schwingungen ergeben dann den tan δ, wie es in den Abbildungen dargestellt ist.

| Messbedingungen: | Temperatur 25°C |
|---|---|
| | Kreisfrequenz 0,1-100 rad/s |
| | Platte/Platte-Konfiguration mit 25 mm Ø |
| | Plattenabstand 1mm |
| | Messgerät: z.B. ARES von Rheometrix Scientific |

Der tan delta gibt also das Verhältnis des elastischen Anteils zum viskosen Anteils der Zubereitung wieder. tan delta = 1 bedeutet somit, die Zubereitung fließt zu 100%, ein tan delta von 0 bedeutet, die Zubereitung kann nicht fließen, sondern ist nur elastisch (z.B. wie ein Gummiband).

### Zum Vergleich und Unterschied.

Eine der bekanntesten Gelstrukturen ist Götterspeise. Hier wirkt Gelantine als Gelbildner. Durch die Weitmaschigkeit die wasserbindenden Gruppen der Gelatine kann eine große Menge an Wasser gebunden werden. Gelbildnernetze schließen das Wasser regelrecht ein. Man spricht von strukturiertem Wasser. Die Wassermoleküle sind manchmal sogar so fest gebunden, dass sie auch unter äußerem Druck nicht abgegeben werden. Deshalb fühlt sich wasserärmeres Hydro-Gel oftmals sogar trocken an.

Solche Gele sind elastisch, wie wir es von der Götterspeise her kennen. Die Strukturen sind nämlich nicht so starr wie in einem Eiskristall angeordnet, sondern flexibel und schwingungsfähig. Auch sind die Wassermoleküle nicht völlig unbeweglich sondern moderat beweglich.

Der Unterschied zu den erfindungsgemäßen, "wobbeligen", Zubereitungen liegt in der hohen Viskoelastizität. D.h. die erfindungsgemäßen Zubereitungen mit hoch elastischer Textur bei geringer Viskosität geben den Schwingungen nach und werden fließfähig.

Götterspeise hingegen wird unter Druck brockig und kann nur stückig gerührt werden.

Die erfindungsgemäßen Zubereitungen erlangen aufgrund der Kombination aus Stearinsäure Emulgator und Polyacrylsäure Verdicker eine überraschend hoch-elastische Textur bei niedriger Viskosität.

Diese erfindungsgemäße Textur und Struktur ermöglicht der Zubereitung auf der Haut ein wässriges, leichtes Hautgefühl zu erbringen, welches sich später in ein pflegend-fettiges Hautgefühl wandeln kann.

Beispielsweise Zusätze wie Stärke und Hautbefeuchtungsmittel werden durch diese strukturellen Eigenschaften so auf der Haut verteilt, das fettige Hautstellen mattiert und trockenen Hautpartien intensiv befeuchtet werden können.

Als Nachweis der beschriebenen Textureigenschaften, "Wobbeligkeit" der O/W-Emulsionen mit stearathaltigen Emulgator und Polyacrylsäure-Verdicker sind nachfolgende Zubereitungen exemplarisch untersucht worden (Gewichtsanteile, in Bezug zur Gesamtmasse der Zubereitungen).

| INCI | Teeso _61 | Teeso _71 | Teeso _72 | Teeso _73 | Teeso _74 | Teeso _75 | Teeso _76 | Teeso _77 |
|---|---|---|---|---|---|---|---|---|
| Methylparaben | 0,3 | 0,3 | 0,3 | 0,3 | 0,3 | 0,3 | 0,3 | 0,3 |
| Propylparaben | 0,1 | 0,1 | 0,1 | 0,1 | 0,1 | 0,1 | 0,1 | 0,1 |
| Stearic Acid | 0 | 0 | 1 | 0 | 0 | 0 | 0 | 0 |
| Aqua | 72,585 | 72,585 | 72,585 | 72,585 | 72,585 | 72,585 | 72,585 | 72,585 |
| Cetearyl Alcohol | 1,5 | 1,5 | 1,5 | 1,5 | 1,5 | 1,5 | 1,5 | 1,5 |
| Glyceryl Stearate + PEG-100 Stearate | 0 | 0 | 0 | 0 | 0 | 0 | 0 | 1 |
| PEG-40 Stearate | 0 | 1 | 0 | 0 | 0 | 0 | 0 | 0 |
| Phenoxyethanol | 0,5 | 0,5 | 0,5 | 0,5 | 0,5 | 0,5 | 0,5 | 0,5 |
| Dimethicone | 1 | 1 | 1 | 1 | 1 | 1 | 1 | 1 |
| Hydrogenated Coco-Glycerides | 2 | 2 | 2 | 2 | 2 | 2 | 2 | 2 |
| Myristyl Myristate | 1 | 1 | 1 | 1 | 1 | 1 | 1 | 1 |
| C12-15 Alkyl Benzoate | 1 | 1 | 1 | 1 | 1 | 1 | 1 | 1 |
| Steareth-21 | 0 | 0 | 0 | 0 | 0 | 0 | 1 | 0 |
| Steareth-2 | 0 | 0 | 0 | 0 | 0 | 1 | 0 | 0 |
| Glycerin | 10 | 10 | 10 | 10 | 10 | 10 | 10 | 10 |
| Sodium Hydroxide (45%) | 0,14 | 0,14 | 0,14 | 0,14 | 0,14 | 0,14 | 0,14 | 0,14 |
| Alcohol Denat. | 5 | 5 | 5 | 5 | 5 | 5 | 5 | 5 |
| Polyglyceryl-3 Distearate | 0 | 0 | 0 | 0 | 1 | 0 | 0 | 0 |
| *Acrylates*/*C10-30 Alkyl Acrylate Crosspolymer* | *0,3* | *0,3* | *0,3* | *0,3* | *0,3* | *0,3* | *0,3* | *0,3* |
| Ethylhexyl Cocoate | 2,5 | 2,5 | 2,5 | 2,5 | 2,5 | 2,5 | 2,5 | 2,5 |
| Glyceryl Stearate Citrate | 1 | 0 | 0 | 0 | 0 | 0 | 0 | 0 |
| Polyglyceryl-3 Methylglucose Distearate | 0 | 0 | 0 | 1 | 0 | 0 | 0 | 0 |
| Tapioca Starch | 0,5 | 0,5 | 0,5 | 0,5 | 0,5 | 0,5 | 0,5 | 0,5 |
| Yogurt Powder | 0,05 | 0,05 | 0,05 | 0,05 | 0,05 | 0,05 | 0,05 | 0,05 |
| CI 17200 | 0,125 | 0,125 | 0,125 | 0,125 | 0,125 | 0,125 | 0,125 | 0,125 |
| Parfum | 0,4 | 0,4 | 0,4 | 0,4 | 0,4 | 0,4 | 0,4 | 0,4 |

### Darin bedeutet:

CI 17200 ist ein Rotfarbstoff (CAS-No.: 3567-66-6) Dinatrium-5-amino-4-hydroxy-3-(phenylazo)naphthalin-2,7-disulfonat und dessen zugelassene Farblacke und Salze.

Joghurtpulver in verschiedenen Säuregraden wird durch Sprühtrocknung aus entrahmter, pasteurisierter Milch gewonnen. Joghurt Pulver besteht meist aus ausgesuchten Milch Fraktionen:
Molke, Molke Konzentrat (Bestandteile des Michserums) und fettfreier Trockenmilch, die mit klassischen Joghurt Bakterien fermentiert wurde.

Joghurtpulver hat meist folgende typische Zusammensetzung, die nachstehend beispielhaft aufgeführt ist. Als Naturprodukt können die Zusammensetzungen jedoch auch Schwanken, insbesondere wenn unteschiedliche Basen zur Gewinnung von Joghurtpulver herangezogen worden sind:
55% Laktose
26% Proteine
7% Milchsäure
5% Mineralien uns Spurenelemente
4% Wasser
2% Lipide
1% Vitamine (A, B1, B2, B,5, B6)

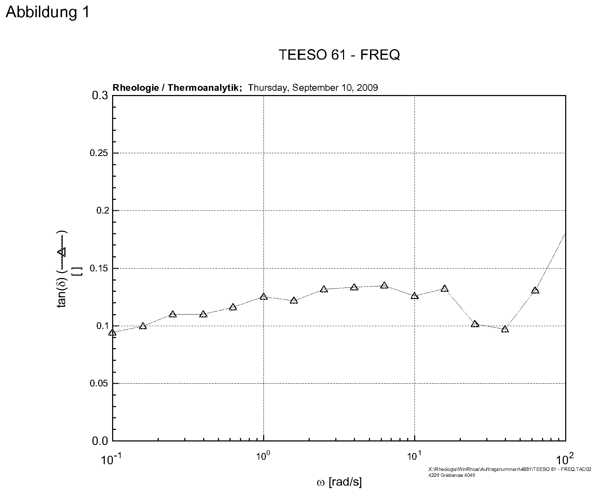

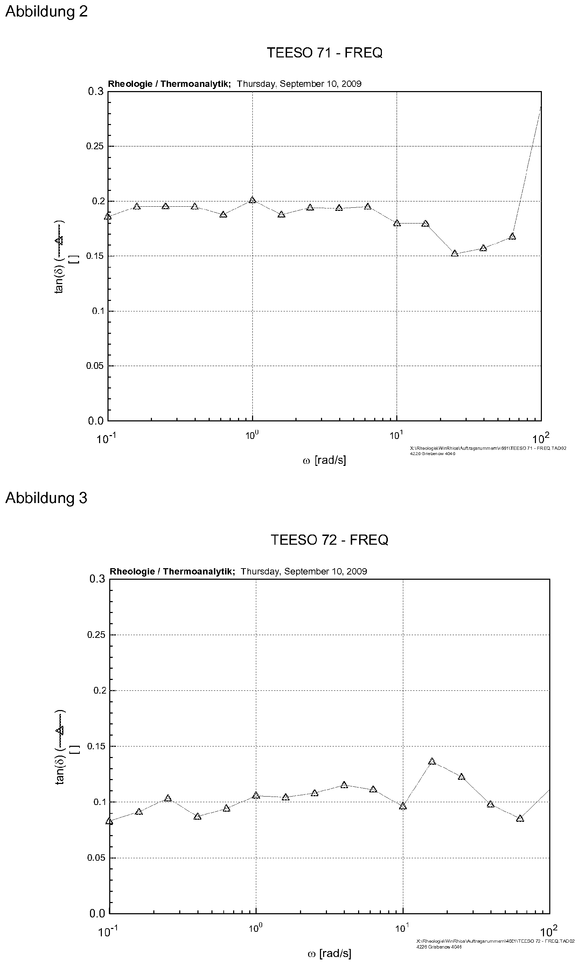

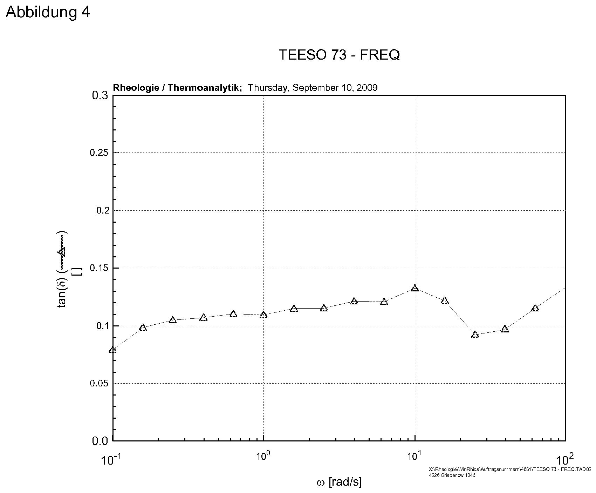

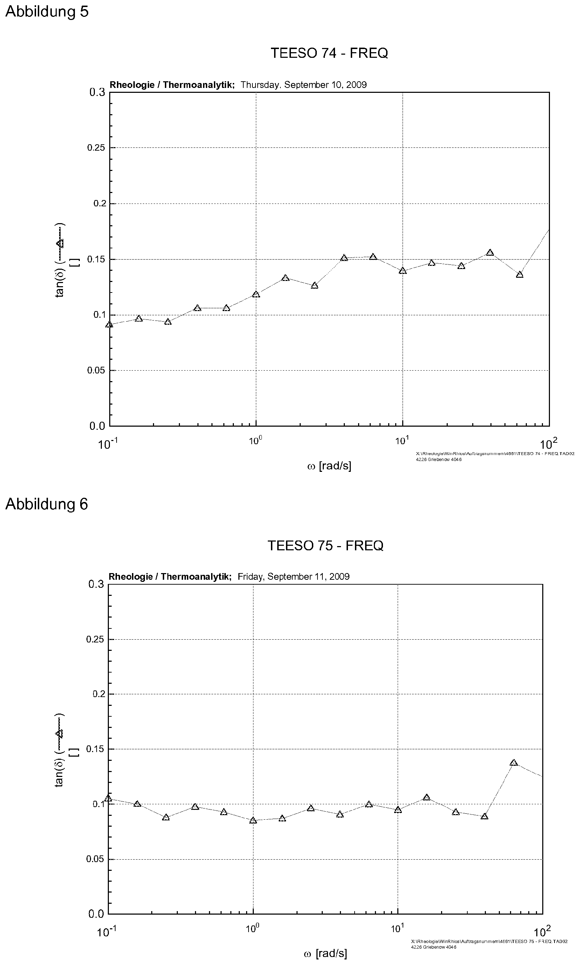

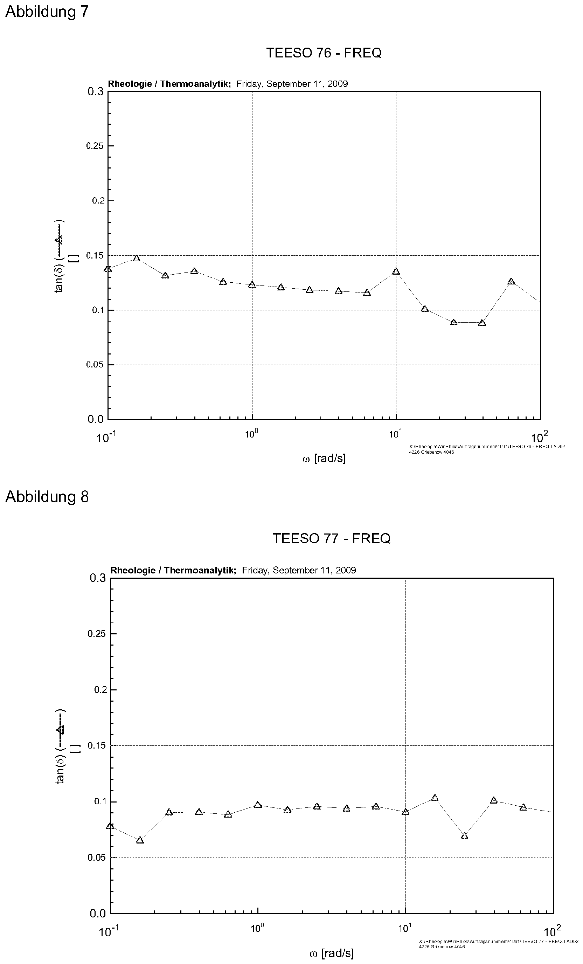

Die Abbildungen 1 bis 7 zeigen die Textureigenschaften der Vergleichszubereitungen.
Abb. 1: Teeso 61 enthält 1% Glycerylstearatcitrat
Abb. 2: Teeso 71 enthält 1% PEG-40 Stearat
Abb. 3: Teeso 72 enthält 1% Stearinsäure
Abb. 4: Teeso 73 mit 1% Polyglyceryl-3 Methylglucose Distearate
Abb. 5: Teeso 74 mit 1% Polyglyceryl-3 Distearat
Abb. 6: Teeso 75 mit 1% Steareth-2
Abb. 7: Teeso 76 mit 1% Steareth-21
Abb. 8: Teeso 77 mit 1% Glyceryl Stearat + PEG-100 Stearat

Der ermittelte tan delta der erfindungsgemäßen Zubereitungen belegt deren viskoelastischen Textureigenschaften.

Die erfindungsgemäßen Zubereitungen auf Basis einer O/W-Emulsion sind vorteilhaft durch die Merkmale charakterisiert:
a.) umfassend ein oder mehrere Stearinsäure-Emulgatoren,
b.) umfassend ein oder mehrere Polyacrylsäure-Verdicker,
c.) Stearinsäure-Emulgator/Polyacrylsäure-Verdicker-Verhältnis von 10 : 1 bis 1:1, insbesondere 3 : 1,
d.) Viskosität 2.000 bis 100.000 mPas, insbesondere im Bereich 8.000 bis 20.000 mPas,
e.) Anteil an Stearinsäure - Emulgatoren maximal 2 Gew.%, insbesondere maximal 1,5 Gew.%,
f.) tan δ im Bereich 0,5 bis 2,5.

Den erfindungsgemäßen Zubereitungen sind vorteilhaft Feuchthaltemittel bzw. sogenannte Moisturizer zugesetzt.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Bevorzugt liegt der Anteil an Hautbefeuchtungsmitteln, auch Moisturizer genannt, im Bereich von 5 bis 60 Gew%, besonders bevorzugt von 8 bis 35 Gew% und ganz besonders bevorzugt von 10-25 Gew%.

Vorteilhafte Moisturizer im Sinne der vorliegenden Erfindung sind insbesondere Glycerin. Weiterhin sind aber Milchsäure, Butylenglykol, Sorbitol, Pyrrolidoncarbonsäure, polymere Moisturizer aus der Gruppe der wasserlöslichen und/oder in Wasser quellbaren und/oder mit Hilfe von Wasser gelierbaren Polysaccharide als Moisturizer zu verwenden. Insbesondere vorteilhaft sind beispielsweise kurzkettige Hyaluronsäure (< 50.000 Dalton) und langkettige Hyaluronsäure (> 50.000 Dalton), Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Den erfindungsgemäßen Zubereitungen sind vorteilhaft Stärke und/oder Joghurtpulver, auch als Yoghurtextrakt zugesetzt.

Als Stärke wird insbesondere Tapiokastärke zugesetzt.

Die beiden Stoffgruppen Stärke oder Joghurtpulver stehen exemplarisch für "fettabsorbierende Stoffe", d.h. sie wirken auf der Haut mattierend und nehmen den Fettglanz.

Die erfindungsgemäßen Zubereitungen lassen sich aufgrund ihrer aussergewöhnlichen Textur aufgrund des Zusatzes von ein oder mehrere Fettabsorbierenden Stoffen zum Mattieren fettiger Hautstellen verwenden.

Ebenso zur Verwendung zum Befeuchtung, insbesondere trockener Hautpartien, sofern Hautbefeuchtungsmittel enthalten sind.

Die kosmetischen und dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Konservierungsmittel, Konservierungshelfer, Bakterizide, Parfüme, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate sofern der Zusatz die geforderten Eigenschaften hinsichtlich der elastischen Textur nicht beeinträchtigt.

Den erfindungsgemäßen Zubereitungen können noch folgende Parfumbestandteile zugesetzt werden, die die "Wobbeligkeit" erstaunlicherweise nicht negativ beeinflussen: (1-oxopropoxy)-,1-(3,3-dimethylcyclohexyl), (süßes) Majoranöl, 1,1-Dimethoxy-2,25-trimethyl-4-hexene, 1,2 Hexandiol, 1,2-Propylene glycol, 1,3,4,6,7,8-Hexahydro-4,6,6,7,8,8-hexamethylcyclopenta-gamma-2-benzopyran, 1,4-Dioxacyclohexadecane-5,16-dione, 1,5,9-Trimethyl-13-oxabicyclo(10.1.0) trideca-4,8-diene, 1,6,7,8-Tetrahydro-1,4,6,6,8,8-hexamthylas-indacen-3<2H>-one, 12-Oxahexadecanolide, 1-Phenyl-3-methyl-3-pentanol, 2,4-Dimethyl-3-cyclohexen-1-carboxaaldehyde, 2,6-Dimethyl-7-octen-2-ol, 2,6-Nonadienal, 2-Acetonapthone-1,2,3,4,5,6,7,8-octahydro-2,3,8,8-tetramethyl, 2-Isobutyl-4-hydroxy-4-methyltetrahydropyran, 2-Methyl-3(4-(2-methylpropyl)phenyl)propanal, 2-Methyl-4-(2,6,6-trimethyl-2(1)-cyclohexen-1-yl)butanal, 2-Methyl-4-phenyl-2-butanol, 2-Methyl-4-phenyl-2-pentanone, 2-Methyl-5-phenylpentan-1-ol, 2-methyl-6-methylene, 2-Methylbutyl acetate, 2-Phenoxyethyl isobutyrate, 2-tert-Pentyl-cyclohexyl acetate, 3(4),8(9)-Dihydroxymethyl tricyclo(5.2.1.0(2,6)decane, 3-(4-t-Butylphenyl)propanal, 3-(5,5,6-Trimethylbicyclo(2.2.1)hept-2-yl)cyclohexan-1-ol, 3,7,11-Trimethyl-1,2,10,-dodecatrien-3-ol(cis & trans), 3,7-Dimethyl-1,3,6-octatriene, 3,7-Dimethyl-1-octanol, 3,7-Dimethyl-2(3),6-nonadienenitrile, 3,7-Dimethyl-2,6-octadien-1-ol, 3,7-Dimethyl-6-octen-1-ol, 3,7-Dimethyloctan-3-yl acetate, 3-Isocamphyl cyclohexanol, 3-Methyl-5-phenyl-1-pentanol, 3-Pentyltetrahydro(2H)pyranyl acetate, 3-Phenyl-1-propanol, 4-(4-Hydroxy-4-methylpentyl)-3-cyclohexene-1-carboxyaldehyde, 4-(p-Hydroxyphenyl)-2-butanone, 4-Acetyl-6-t-butyl-1,1-dimetylindane, 4-Carvomenthenol, 4-t-Butylcyclohexyl acetate, 5-Phenylpentanol, 7-Acetyl-1,1,3,4,4,6-hexamethyltetralin, 7-Octen-2-ol, Abietyl acetate, Acetic acid, Acetyl Hexamethyl Tetralin (Moschus-Verb.), Acetyl Trifluoromethylphenyl Valylglycine, Acrylamidammoniumacrylatcopolymer, Allyl phenoxyacetate, alpha-Amylcinnamaldehyde, alpha-Butylcinnamaldehyde, alpha-Hexylcinnamaldehyde, alpha-Ionone, alpha-Isomethylionone, alpha-Methyionantheme, alpha-Methyl-3,4-methylene dioxyhydrocinnamic aldehyde, alpha-Methylbenzyl acetate, alpha-Pinene, alpha-Terpineol, Aluminummagnesiumhydroxidstearat, Amaranthus Extract, Ammoniumlactat, Ammoniumpolyacrylat, Ammoniumpolyacryloyldimethyltaurat, Amylacetat, Amylsalicylat, Andrographolide, Anethol, Anisic acid, Anisöl, Anisyl acetate, Anisyl alcohol, Annatto, Anthemis Nobilis Flower, Arginin PCA, Ascorbyl Tocopheryl Maleate, Avena Aqua, Bayöl, Benzaldehyde, Benzyl acetate, Benzyl alcohol, Benzyl benzoate, Benzyl Cinnamate, Benzyl laurate, Benzyl salicylate, Bergamot oil, Bergamot oil, bergaptene free, beta-Caryophyllene, beta-Ionone, beta-Pinene, BGT, Biosaccharide Gum 3, Bois de rose oil, Bornyl acetate, Butyl Hydroquinone, Butylated hydroxytoluene, Calendulaöl, Camphene, Capryloylsalicylsäureester, Carnaubau Wax, Carvon , Cassia Alata, Cedarwood oil, Cedrenol, Cedrenyl acetate, Cedrol, Cedrol methyl ether, Cedryl acetate, Chlorhexidine Digluconate, Chrysanthenum Parthenium, Cinnamic alcohol, Cinnamic aldehyde, cis-3-Hexenyl salicylate, Citral, Citronella oil, Citronellal, Citronellyl acetate, Citrus Aurantium Dulcis Blossoms, Citrus oil distilled, Clove bud oil, Cocoglucosid, Coffea Robusta, Cognac oil, Coleus Barbatus Extract, Coumarin, Cyclamen aldehyde, Cyclohexyl salicylate, d-Camphor, Decanal, delta-3-Caren, Diacetin, Diethyl malonate, Diethyl phthalate, Diethylene glycol monoethyl ether, Dihydromyrcenyl acetate, Dihydroterpinyl acetate, Dimethoxy Di-p-Cresol, Dimethyl benzyl carbinyl acetate, Dimethyl benzyl carbinyl butyrate, Dimethyl phthalate, Dimethylbrassylat, Dinatriumadenosintriphosphat, Dipenten , Diphenyl ether, Diphenyldimethicon, Dipropylene glycol, Disodium NADH, Disteardimoniumhectorit, d-Limonene, Natriumsuccinat, Dodecene, Echium Lycopsis Oil, Eclipta Prostrata Extract, Eperua Falcata , Erythritol, Essential Oils, Ethyl acetate, Ethyl acetoacetate, Ethyl Bisiminomethylguaiacol Manganese Chloride, Ethyl butyrate, Ethyl Glucoside, Ethyl maltol, Ethyl vanillin, Ethyl-2methyl-1,3-dioxolane-2-acetate, Ethylene brassylate, Ethyllinalool, Ethyllinylyl acetate, Ethylvanillin, Etyhl methylphenylglycidate, Eucalyptol, Eucalyptus oil, Eugenol , Extrakt aus Blättern der Melisse, Fir needle oil, Formaldehyde cyclododecyl ethyl acetal, Galbanum oil, gamma-Decalactone, gamma-Nonalactone, gamma-Terpinene, gamma-Undecalactone, Gartenrautenöl, Geranium oil, Geranyl acetate, Glucosylrutin, Glyceryl caprylate, Grapefruit oil, Guanosine, Gum Benzoin, Heliotropin, Heptapeptide-6, Hexahydro-4,7-methanoinden-5(6)-yl acetate, Hexyl acetate, Hexyl alcohol, Hexyl salicylate, Hexylene glycol, Hierochloe Odorata Extract, Holunderextrakt, Holunderöl, Hopfenöl, Humulus Japonicus Flower / Leaf / Stem Extract, Hydrated Magnesium Silicate, Hydrierte Palmenkernglyceride, hydriertes Palmenglycerid, hydriertes Palmenglyceridcitrat, Hydrogenated Myristyl Olive Esters, Hydrolisiertes Weizenprotein PG-Propylmethylsilandiol, Hydrolyzed Cera Alba, Hydrolyzed Wheat Protein PG-Propyl Methylsilanediol, Hydroxycitronellal, Hydroxycitronellol, Hydroxyethylacrylat / Natriumacryloyldimethyltauracopolymer, Hydroxyethylpiperazine Ethane Sulfonic Acid, Hydroxypropyl Tetrahydropyrantriol, Ingweröl, lonone, Isoamyl salicylate, Isoamylacetat, Isobornyl acetate, Isobornylcyclohexanol, Isobutyl salicylate, Isodeceth-6, Isoeugenol, Isopropyl myristate, Isoquercitrin, Juniper berry oil, Kaempferia Galanga Root Extract, Kamelienöl, Kamillenöl, Kampher, Kardamomöl, Kiefern(teer)öl, Kiefernöl, Kreuzkümmelextrakt, Kümmelöl, Kupfergluconat, Labdanum oil, Lagerstroemia Indica Extract, Lauric aldehyde, Lauryl alcohol, Lauryl Dimonium Hydroxypropyl Hydrolyzed Soy Protein, Lauryl PolyglucosideSodium Hydroxypropyl Starch Phosphate, Lavandin oil, Lavender oil, Lemon oil, Lemon oil terpenes, Lemon terpenes, Lemongrassöl, Liebstöckelöl, Lime oil, Linalool, Linalyl acetate, Linseed Acid, Linseed Acid Magnesiumaspartat, I-Menthol, Lorbeeröl, Lupinus Luteus Extract, Majantol, Maltol isobutyrate, Mandarin oil, Melibiose, Menthanyl acetate, Menthol, Menthol liquid, Menthone, Menthyl acetate, Menthyllactat, Menthylsalicylat, Methyl 2-nonenoate, Methyl abietate, Methyl cedryl ketone, Methyl dihydrojasmonate, Methyl ester of rosin, Methyl Rosinate, Methyl-alpha-ionone, Methyl-delta-ionone, Methyldihydrojasmonate, Methyleugenol, Methylpentenolone, Methylserine, Methylsilanol / Silicate CrosspolymerAmaranthus Caudatus Extract, Mineral oil, Musk ketone, Muskatnussöl, Myrcene, Myrcenyl acetate, Myristicin, Natriummetabisulfit, Nelkenöl, Nelkenblattöl, Nerol, Neroli bigarade oil, Nerolidol, Neryl acetate, Nicotinamide, Nopyl acetate, Nutmeg oil, Nylon-66, Ocotea Cymbarum Öl, Octanal, Octyl acetate, Octyldodecanol, Öl aus Blättern der Melisse, Oleyl Erucate, omega-Pentadecalactone, Orange oil, Orange terpenes, Orange terpenes (natural), Orangenblütenöl, Orangenblütenwasser, Orangenextrakt, Orangenschalenextrakt, o-t-Butylcyclohexyl acetate, Oxacyclohexadecen-2-one, Oxothiazolidinecarboxylic Acid, Oxothiazolidinecarboxylsäure, Palmitoyl Lysyl Aminovaleroyl Lysine, Palmitoyl Tetrapeptide-10, Palmitoylpentapeptide 4, Patchouli oil, p-Cymene, PEG / PPG-14/7 Dimethyl Ether, PEG-10 Dimethicone / Vinyl Dimethicone Crosspolymer, PEG-2 Stearyl Ether, PEG-20 Stearyl Ether, PEG-24 Cetyl Ether, PEG-24 Cholesteryl Ether, PEG-60 Glyceryl Isostearate, PEG-8 Laurat, Penethyl Alcohol, Pentadecalacton, Penthylene Glycol, Peppermint oil, Petersilienöl, Petitgrain oil, Pfefferminzextrakt, Pfefferminzöl, Phenethyl alcohol, Phenoxyethanol, Phenylacetaldehyde glyceryl acetal, Phenylethyl acetate, Phenylpropanol, Phytol, Pisces Collagen, Platanus Occidentalis, p-Methylanisole, Poly C10-30 Alkyl Acrylate, Polyacrylate-13, Polyacrylate-3, Polycaprolactone, Polyester-5, Polyglyceryl-2 Caprate, Polyglyceryl-6 Polyricinoleate zugesetzt sein., p-t-Butyl-alpha-methyldihydrocinnamic aldehyde, Pulegone, Punica Granatum Fruit Juice, Pyracantha Fortuneana Extract, Pyrus Malus Water, Rhodinol, Rosemary oil, Rosenextrakt, Rosenöl, Rosmarinöl, Sabinene, Salbeiöl, Sandalwood oil, Sandelholzöl, Sarcosin, Sassafrasbaumöl, Saxifraga Sarmentosa Extrakt, Schafgarbenöl, Sclareoate, Scutellaria Baicalensis Extrakt, Selaginella Tamariscina Extract, Shorea Robusta Butter, Sodium Dodecylbenzene Sulfonate, Sodium Hydroxyethyl Acrylate / Acryloyldimethyl Taurate Copolymer, Sodium Mannose Phosphate, Sojaisoflavon, Spearmint oil, Spike lavender oil, Stearic acid, Succinoglycan, Sucrose Polycottonseedate, Teebaumöl, Teeröl, Terpineol, Terpineol, Terpinyl acetate, Tetrahydrobisdemethoxycurcumin, Tetrahydrobisdemethoxydiferuloylmethane, Tetrahydrodemethoxycurcumin, Tetrahydrodemethoxydiferuloylmethane, Tetrahydrolinalool, Tetrahydromyrcenol, Thiodipropionic Acid, Thiotaurine, Thyme oil, Thymianöl, Thymol, Tocopheryl Glucoside, Tocopherylglucosid, trans-Anethole, trans-beta-Ionone, Treemoss abs., Tri C14-15 Alkylcitrate, Triacetin, Trichloromethyl phenyl carbinyl acetate, Tricyclodecenyl propionate, Trideceth-6, Triethyl citrate, Trioctyldodecyl Citrate, Trioxaundecanedioic Acid, Undecanal,2-methyl-, Vanille, Vanillin, Vetiver oil, Wacholderteer, Weihrauchharz, Weihrauchharzextrakt, Xymenynic Acid, Ylang ylang oil, Zimtöl, Zimtsäure, Zinkgluconat, Zitronengrasöl, Zitronenöl,

Die erfindungsgemäßen Zubereitungen sind topisch applizierbar formuliert. Unter topisch applizierbaren Zubereitungen werden erfindungsgemäß Lotionen, Gele, Emulsionen, Aerosol-Mousse, pumpbare Zubereitungen, Cremes sowie damit getränkte oder beladene Tücher, Pads oder Pflaster verstanden.

Die nachfolgenden Beispiele zeigen kosmetische Zubereitungen mit uneingeschränkter Wirkleistung und hochelastischer Textur bei niedriger Viskosität.

Die Zahlenangaben sind Gewichtsanteile bezogen auf die Gesamtmasse der Zubereitung.

### Beispiele

**O/W-Emulsion**

| | 1 | 2 | 3 | 4 | 5 |
|---|---|---|---|---|---|
| Glycerylstearatcitrat | 1,0 | 1,1 | 0,75 | 1,09 | 0,45 |
| Tapiokastärke | 0,5 | 6,0 | 1,0 | 3,0 | --- |
| Myristylmyristat | 1,0 | --- | --- | --- | 3,0 |
| Triclosan | --- | --- | 0,06 | --- | --- |
| C12-15 Alkylbenzoat | 1,0 | 3,0 | 3,0 | 3,0 | --- |
| Ethylhexylcocoat | 2,5 | --- | --- | --- | 4,0 |
| lineares Silikonöl | 1,0 | 1,0 | 5,0 | 1,0 | 2,0 |
| Propylparaben | 0,2 | 0,1 | 0,1 | 0,1 | 0,2 |
| hydrierte Kokosglyceride | 2,0 | --- | --- | --- | 0,8 |
| Yoghurtextrakt | 0,05 | --- | --- | --- | 1,0 |
| Cetearylalkohol | 1,5 | 2,0 | 2,0 | 3,0 | 0,8 |
| Phenoxyethanol | 0,5 | 0,5 | 0,5 | 0,5 | 0,6 |
| Glycerin | 10,0 | --- | --- | --- | 6,5 |
| Polyacrylsäure, Natrium-Salz | 0,3 | 0,367 | 0,25 | 0,31 | 0,15 |
| Xanthan Gum | --- | --- | --- | --- | 0,2 |
| Methylparaben | 0,3 | 0,25 | 0,3 | 0,4 | 0,3 |
| Alkohol | 5,0 | --- | --- | --- | 3,0 |
| Parfum | 0,4 | 0,3 | 0,3 | 0,3 | 0,6 |
| Farbstoff Rot CI 17200 | 0.0001 | 0,005 | 0,035 | 0,05 | 0,00015 |
| Wasser | ad 100 | ad 100 | ad 100 | ad 100 | ad 100 |

**O/W-Emulsion**

| | 6 | 7 | 8 | 9 | 10 |
|---|---|---|---|---|---|
| PEG-40 Stearat | 0,3 | 0,9 | 1,1 | 0,3 | 0,6 |
| Tapiokastärke | 3,0 | 6,0 | 1,0 | 3,0 | 0,15 |
| Caprylic/Capric Triglycerid | 3,1 | 3,0 | 4,0 | 3,0 | --- |
| medizinisches Weißöl | 6,0 | --- | 4,0 | --- | 2,0 |
| lineares Silikonöl | 2,8 | 1,0 | 5,0 | 1,0 | 2,0 |
| Propylparaben | 0,1 | 0,11 | 0,15 | 0,1 | 0,2 |
| Glycerylstearat | 0,7 | --- | --- | --- | 0,9 |
| Yoghurtextrakt | 0,05 | --- | --- | --- | 1,0 |
| Cetylalkohol | 1,5 | 2,0 | 4,0 | 3,0 | 0,9 |
| Phenoxyethanol | 0,5 | 0,5 | 0,5 | 0,4 | 0,6 |
| Glycerin | 5,0 | --- | 3,5 | 15,0 | 6,5 |
| Polyacrylsäure, Natrium-Salz | 0,3 | 0,3 | 0,367 | 0,1 | 0,5 |
| Glycerylglucose | 6,0 | --- | --- | --- | 0,2 |
| Methylparaben | 0,3 | 0,25 | 0,3 | 0,4 | 0,3 |
| C13-16 Isoparaffin | 2,9 | --- | 1,8 | --- | 3,0 |
| Parfum | 0,4 | 0,3 | 0,15 | 0,3 | 0,6 |
| Farbstoff Rot D+C No. 33 CI 17200 | 0.0002 | 0,005 | 0,035 | 0,045 | 0,00015 |
| Wasser | ad 100 | ad 100 | ad 100 | ad 100 | ad 100 |

## Patentansprüche

1. Kosmetische oder dermatologische Zubereitung auf Basis einer O/W-Emulsion umfassend ein oder mehrere Stearinsäure-Emulgatoren und ein oder mehrere Polyacrylsäure-Verdicker **dadurch gekennzeichnet, dass**
der oder die Stearinsäure-Emulgatoren gewählt werden aus der Gruppe Stearinsäure, Glycerylstearatcitrat, Polyglyceryl-3 Methylglucose Distearate, Polyglyceryl-3 Distearat, Steareth-21, Steareth-2 , PEG-40 Stearat und Glyceryl Stearate + PEG-100 Stearate,
das Verhältnis von Stearinsäure-Emulgatoren zu Polyacrylsäure-Verdicker im Bereich 10 : 1 bis 1 : 1 liegt,
der Anteil an Stearinsäure - Emulgatoren auf maximal 1,5 Gew.% begrenzt ist, bezogen auf die Gesamtmasse der Zubereitung,
die Zubereitung eine Viskosität im Bereich von 12.000 bis 100.000 mPas aufweist und
die Zubereitung einen tan δ im Bereich von 0,05 bis 0,25 aufweist; beide Parameter wie in der Beschreibung offenbart.

2. Zubereitung nach einem der vorstehenden Ansprüche, **dadurch gekennzeichnet, dass** als Polyacrylsäure-Verdicker ACRYLATES/C10-30 ALKYL ACRYLATE CROSSPOLYMER gewählt wird.

3. Zubereitung nach einem der vorstehenden Ansprüche, **dadurch gekennzeichnet, dass** die Zubereitung Joghurtpulver,- extrakt enthält.

4. Zubereitung nach einem der vorstehenden Ansprüche , **dadurch gekennzeichnet, dass** die Zubereitung Stärke enthält.

5. Zubereitung nach einem der vorstehenden Ansprüche, **dadurch gekennzeichnet, dass** die Zubereitung ein oder mehrere Hautbefeuchtungsmittel enthält.

6. Zubereitung nach einem der vorstehenden Ansprüche, **dadurch gekennzeichnet, dass** die Zubereitung ein oder mehrere Parfumbestandteile enthält.

7. Verwendung von Zubereitungen nach einem der vorstehenden Ansprüche, umfassend ein oder mehrere Fettabsorbierende Stoffe zum Mattieren fettiger Hautstellen.

8. Verwendung von Zubereitungen nach einem der vorstehenden Ansprüche, umfassend ein oder mehrere Hautbefeuchtungsmittel zum Befeuchtung, insbesondere trockener Hautpartien.

9. Verwendung von ein oder mehreren Stearinsäure-Emulgatoren und ein oder mehrere Polyacrylsäure-Verdickern, **dadurch gekennzeichnet, dass** der oder die Stearinsäure-Emulgatoren gewählt werden aus der Gruppe Stearinsäure, Glycerylstearatcitrat, Polyglyceryl-3 Methylglucose Distearate, Polyglyceryl-3 Distearat , Steareth-21, Steareth-2 , PEG-40 Stearat und Glyceryl Stearate + PEG-100 Stearate und das Verhältnis von Stearinsäure-Emulgatoren zu Polyacrylsäure-Verdicker im Bereich 10 : 1 bis 1 : 1 zur Herstellung einer kosmetischen oder dermatologischen Zubereitung auf Basis einer O/W-Emulsion mit einem tan δ im Bereich von 0,05 bis 0,25 und einer Viskosität im Bereich zwischen 12000-100.000 mPas, wobei der Anteil an Stearinsäure-Emulgatoren auf maximal 1,5 Gew.% begrenzt ist, bezogen auf die Gesamtmasse der Zubereitung und die beide Parameter wie in der Beschreibung offenbart sind.

## Claims

1. Cosmetic or dermatological preparation based on an O/W emulsion comprising one or more stearic acid emulsifiers and one or more polyacrylic acid thickeners **characterized in that**
the stearic acid emulsifier(s) are selected from the group comprising stearic acid, glyceryl stearate citrate, polyglyceryl-3 methylglucose distearate, polyglyceryl-3 distearate, steareth-21, steareth-2, PEG-40 stearate and glyceryl stearate + PEG-100 stearate,
the ratio of stearic acid emulsifiers to polyacrylic acid thickeners is in the range of 10:1 to 1:1,
the proportion of stearic acid emulsifiers is restricted to a maximum of 1.5% by weight, based on the total mass of the preparation,
the preparation has a viscosity in the range of 12 000 to 100 000 mPas and
the preparation has a tan δ in the range of 0.05 to 0.25; both parameters as disclosed in the description.

2. Preparation according to any of the preceding claims, **characterized in that** the polyacrylic acid thickener selected is acrylates/C10-30 alkyl acrylate crosspolymer.

3. Preparation according to either of the preceding claims, **characterized in that** the preparation comprises yoghurt powder extract.

4. preparation according to any of the preceding claims, **characterized in that** the preparation comprises starch.

5. Preparation according to any of the preceding claims, **characterized in that** the preparation comprises one or more skin moisturizing agents.

6. Preparation according to any of the preceding claims, **characterized in that** the preparation comprises one or more perfume constituents.

7. Use of preparations according to any of the preceding claims comprising one or more fat-absorbing substances for matting greasy skin areas.

8. Use of preparations according to any of the preceding claims comprising one or more skin moisturizing agents for moisturizing particularly dry areas of skin.

9. use of one or more stearic acid emulsifiers and one or more polyacrylic acid thickeners, **characterized in that** the stearic acid emulsifier(s) are selected from the group comprising stearic acid, glyceryl stearate citrate, polyglyceryl-3 methylglucose distearate, polyglyceryl-3 distearate, steareth-21, steareth-2, PEG-40 stearate and glyceryl stearate + PEG-100 stearate and the ratio of stearic acid emulsifiers to polyacrylic acid thickeners is in the range of 10:1 to 1:1, for preparing a cosmetic or dermatological preparation based on an O/W emulsion having a tan δ in the range of 0.05 to 0.25 and a viscosity in the range of 12 000-100 000 mPas, wherein the proportion of stearic acid emulsifiers is restricted to a maximum of 1.5% by weight, based on the total mass of the preparation, and both parameters are as disclosed in the description.

## Revendications

1. Préparation cosmétique ou dermatologique à base d'une émulsion huile/eau comprenant un ou plusieurs émulsifiant(s) de type acide stéarique et un ou plusieurs épaississant(s) de type poly(acide acrylique), **caractérisée en ce que**
le ou les émulsifiant(s) de type acide stéarique est/sont choisi(s) dans le groupe de l'acide stéarique, du stéarate-citrate de glycéryle, du, méthylglucosedistéarate de polyglycéryle-3, du distéarate de polyglycéryle-3, du stéareth-21, du stéareth-2, du stéarate de PEG-40 et du stéarate de glycéryle + stéarate de PEG-100,
le rapport d'émulsifiants de type acide stéarique à épaississant(s) de type poly(acide acrylique) se situe dans la plage de 10:1 jusqu'à 1:1,
la part d'émulsifiants de type acide stéarique est limitée à 1, 5% en poids au maximum, par rapport à la masse totale de la préparation,
la préparation présente une viscosité dans la plage de 12.000 jusqu'à 100.00 mPa.s et
la préparation présente une valeur de tan δ dans la plage de 0,05 jusqu'à 0,25 ; les deux paramètres étant tels que divulgués dans la description.

2. Préparation selon l'une quelconque des revendications précédentes, **caractérisée en ce que** le polymère réticulé d'acrylates/acrylate d'alkyle en C₁₀₋₃₀ est choisi en tant qu'épaississant de type poly(acide acrylique).

3. préparation selon l'une quelconque des revendications précédentes, **caractérisée en ce que** la préparation contient de la poudre ou de l'extrait de yaourt.

4. Préparation selon l'une quelconque des revendications précédentes, **caractérisée en ce que** la préparation contient de l'amidon,

5. Préparation selon l'une quelconque des revendications précédentes, **caractérisée en ce que** la préparation contient un ou plusieurs agent(s) d'hydratation de la peau.

6. Préparation selon l'une quelconque des revendications précédentes, **caractérisée en ce que** la préparation contient un ou plusieurs ingrédients de parfum.

7. Utilisation de préparations selon l'une quelconque des revendications précédentes, comprenant une ou plusieurs substance(s) absorbant les graisses afin de matifier des zones cutanées grasses.

8. Utilisation de préparations selon l'une quelconque des revendications précédentes, comprenant un ou plusieurs agent(s) d'hydratation de la peau pour l'hydratation, en particulier de parties de la peau plus sèches.

9. Utilisation d'un ou plusieurs émulsifiant(s) de type acide stéarique et d'un ou plusieurs épaississant (s) de type poly(acide acrylique), **caractérisée en ce que** le ou les émulsifiant(s) de type acide stéarique est/sont choisi(s) dans le groupe de l'acide stéarique, du stéarate-citrate de glycéryle, du méthylglucosedistéarate de polyglycéryle-3, du distéarate de polyglycéryle-3, du stéareth-21, du stéareth-2, du stéarate de PEG-40 et du stéarate de glycéryle + stéarate de PEG-100 et le rapport des émulsifiants de type acide stéarique aux épaississants de type poly(acide acrylique) se situe dans la plage de 10:1 jusqu'à 1:1, pour la préparation d'une préparation cosmétique ou dermatologique à base d'une émulsion huile/eau présentant une valeur de tan δ dans la plage de 0,05 jusqu'à 0,25 et une viscosité dans la plage entre 12.000-100.00 mPa.s, la part d'émulsifiants de type acide stéarique étant limitée à 1,5% en poids au maximum, par rapport à la masse totale de la préparation et les deux paramètres étant tels que divulgués dans la description.
